# EUROPEAN PATENT SPECIFICATION

(11) **EP 3 817 728 B1**
(45) Date of publication and mention of the grant of the patent: **22.05.2024**
(21) Application number: 19736304.7
(22) Date of filing: 03.07.2019
(51) Int. Cl.: A61K 9/20, A61K 31/4196

(54) **PHARMACEUTICAL COMPOSITION COMPRISING AN IRON CHELATING AGENT AND METHOD FOR THE PREPARATION THEREOF**
PHARMAZEUTISCHE ZUSAMMENSETZUNG MIT EINEM EISENCHELATBILDNER UND VERFAHREN ZUR HERSTELLUNG DAVON
COMPOSITION PHARMACEUTIQUE COMPRENANT UN AGENT CHÉLATEUR DE FER ET SON PROCÉDÉ DE PRÉPARATION

(30) Priority: 03.07.2018 GR 20180100306
(43) Date of publication of application: 12.05.2021
(73) Proprietor: Pharmathen S.A., 15351 Pallini Attikis (GR)
(72) Inventor: KARAVAS, Evangelos, 153 51 Pallini Attikis (GR); KOUTRIS, Efthymios, 153 51 Pallini Attikis (GR); SAMARA, Vasiliki, 153 51 Pallini Attikis (GR); KOUTRI, Ioanna, 153 51 Pallini Attikis (GR); KALASKANI, Anastasia, 153 51 Pallini Attikis (GR); KIZIRIDI, Christina, 153 51 Pallini Attikis (GR); KAKOURIS, Andeas, 153 51 Pallini Attikis (GR)
(86) International application number: PCT/EP2019/025208
(87) International publication number: WO 2020/007505

(56) References cited:
- WO-A1-2010/035282
- WO-A1-2018/007956
- WO-A1-2018/059922
- US-A1- 2018 071 220

## Description

### TECHNICAL FIELD OF INVENTION

The present invention relates to a stable pharmaceutical formulation for oral administration containing a therapeutically effective quantity of an iron chelating agent such as Deferasirox and a method for the preparation thereof.

### BACKGROUND OF THE INVENTION

Iron overload is an excess iron in the body. Even in mild cases of iron overload, it increases the risk for liver disease (cirrhosis, cancer), heart attack or heart failure, diabetes mellitus, osteoarthritis, osteoporosis, metabolic syndrome, hypothyroidism, hypogonadism and in some cases premature death. Iron overload is a serious chronic condition that must be properly diagnosed and treated.

The major cause of chronic iron overload is regular blood transfusions, which can be required for managing many chronic health conditions. While removal of excess iron by repeated phlebotomy is the primary treatment; patients with transfusion-induced iron overload are already anemic so therapeutic phlebotomy is not usually an option. Iron chelation drug therapy, on the other hand, has been shown to benefit patients who have transfusion-dependent anemias.

Deferasirox is a synthetic, orally bioavailable, tridentate triazole derived from salicylic acid with iron-chelating activity. Deferasirox chelates iron at a 2:1 (ligand:iron) ratio. Because of its oral availability and long plasma half-life, this agent may be superior to the older desferrioxamine which is orally inactive and has a short plasma half-life. Its main use is to reduce chronic iron overload in patients who are receiving long-term blood transfusions for conditions such as beta-thalassemia and other chronic anemias.

The chemical name of Deferasirox is 4-[3,5-Bis (2-hydroxyphenyl)-1H-1,2,4-triazol-1-yl]-benzoic acid The molecular formula is C₂₁H₁₅N₃O₄ corresponding to a molecular weight of 373.4. It is a white to slightly yellow powder. Deferasirox is soluble in dimethyl sulfoxide, in dimethylformamide and is insoluble in water. It is classified as a Class II drug (poorly soluble, highly permeable) according to the Biopharmaceutics Classification System (BCS).

Deferasirox is crystalline and exhibits polymorphism. The crystal Form-A used in the present invention is disclosed in WO2008/065123.

WO2009/106824 discloses an oral pharmaceutical formulation comprising an active ingredient such as Deferasirox in combination with one or more pharmaceutical adjuvants, wherein the active ingredient, or combination of the active ingredient and the or each adjuvant, is coated with water soluble polymer or combination of water soluble and water insoluble polymer.

US2009/142395 discloses a process of preparing Deferasirox formulations having sufficiently high dissolution rate and good bioavailability wherein said process comprises co-milling active ingredient with at least two pharmaceutically acceptable excipients in the absence of any solvent.

Although each of the patents above represents an attempt to provide stable Deferasirox compositions for oral administration, there still remains the need in the art for alternative formulations with enhanced dissolution and absorption properties leading to better bioavailability.

### SUMMARY OF THE INVENTION

It is, therefore, an object of the present invention to provide a thermodynamically stable and efficient product comprising an iron chelating agent such as Deferasirox suitable for oral administration.

The present invention aims at developing a formulation that not only matches the physical and chemical attributes of the reference product but also overcomes the disadvantages associated with the prior art compositions.

Further object of the present invention is to provide an immediate release film-coated tablet comprising Deferasirox as an active ingredient, which is bioavailable and with sufficient self-life.

Yet another object of the present invention is to provide a solid dosage form for oral administration containing Deferasirox which can be formulated into dosage forms of different strengths by proportionally adjusting the amounts of the pharmaceutically acceptable excipients, as well as the active compound Deferasirox.

It is another object of the present invention to provide a stable solid dosage formulation for oral administration containing Deferasirox that overcomes the poor water solubility of the active ingredient and has acceptable pharmacotechnical properties.

A major object of the present invention is the selection of the optimal combination of pharmaceutical acceptable excipients, the effective drug substance particle size distribution and the method of preparation of final product in order to achieve the appropriate dissolution profile and stability for the finished dosage form. Said dosage form affords predictable and reproducible drug release rates in order to achieve better treatment to a patient.

A further approach of the present invention is to provide a tablet composition for oral administration comprising Deferasirox which is manufactured through a fast, simple and cost-effective process.

According to another embodiment of the present invention, a process for the preparation of a solid dosage form for oral administration, containing an iron chelating agent and in particular Deferasirox as an active ingredient and an effective amount of a non-ionic surfactant is provided, which comprises the following steps:
- Wet granulation of internal phase excipients and API using water as kneading solvent;
- Drying;
- Mixing with slugging phase;
- Compressing into slugs;
- Milling the resulted slug to yield the granules;
- Mixing with external phase excipients;
- Tableting;
- Optionally applying a film-coating.

Other objects and advantages of the present invention will become apparent to those skilled in the art in view of the following detailed description.

### DETAILED DESCRIPTION OF THE INVENTION

For the purposes of the present invention, a pharmaceutical composition comprising an active ingredient is considered to be "stable" if said ingredient degrades less or more slowly than it does on its own and/or in known pharmaceutical compositions.

As already mentioned the main object of the present invention is to provide a stable pharmaceutical composition of Deferasirox for oral administration that is simple to manufacture, bioavailable, cost effective and possesses good pharmacotechnical properties.

High drug loading formulations have recently been drawing increased attention due to patient preferences for smaller dosage size. Maximization of drug loading can effectively decrease dosage form sizes as well as reduce manufacturing batch sizes to provide cost savings. Moreover, high drug loading is a requirement for active ingredients with poor water solubility to achieve desired therapeutic effects. Typically, active ingredient powders have relatively small particle sizes with non-spherical particle shape. They usually possess high cohesivity and poor flowabiliy. In many cases, direct compaction and roller compaction technologies have difficulties in processing formulations when drug loading exceeds 50%. Wet granulation is the preferred choice for processing high drug loading formulations because of its process robustness and consistency in particle dispersion and granulation. According to the present invention Deferasirox is present in an amount higher than 60% by weight based on the total weight of the medicament.

Solubility, the phenomenon of dissolution of solute in solvent to give a homogenous system, is one of the important parameters to achieve desired concentration of drug in systemic circulation for desired (anticipated) pharmacological response. Deferasirox belongs to class II according to the Biopharmaceutical Classification System. It is permeable but relatively insoluble, and is considered not such good clinical candidate without the use of enhanced formulation techniques aimed at increasing solubility or rate of dissolution. Solubility enhancement is a major challenge for formulation development. Any drug to be absorbed must be present in the form of solution at the site of absorption. Various techniques are used for the enhancement of the solubility of poorly soluble drugs which include physical and chemical modifications of drug and other methods like particle size reduction, crystal engineering, salt formation, solid dispersion, use of surfactant, complexation, and addition of alkalizing or acidifying agents. Selection of solubility improving method depends on drug property, site of absorption, and required dosage form characteristics.

The particle size of the API is a critical parameter that can affect the solubility of low solubility APIs such as Deferasirox. The specific surface area is increased with decreasing particle size of the drug, resulting in an increase in dissolution rate. In most circumstances the dissolution rate of poorly soluble drugs is strongly related to the particle size distribution and thus the dissolution profile of the final product.

In order for a drug to have its effect after oral administration it must go into solution and then diffuse through the gut wall into the body. The first step in that process is the disintegration of the dosage form followed by dissolution of the active ingredient. One way to increase dissolution rate of poorly soluble drugs such as Deferasirox is to increase the surface available for dissolution by reducing particle size. It has been surprisingly found that the objects of the present invention are achieved when the formulation is prepared using Deferasirox in micronized form with D90<10µm.

The addition of surfactants is another technique by which solubility may be increased or stability improved in a formulation. Because of their amphiphilic structure, surfactants contain both hydrophilic and hydrophobic groups that allows for the reduction in the interfacial tension between solid particles and liquid, thus improving solubility. Non-ionic surfactants are more widely used in the pharmaceutical industry as compared with anionic, cationic or amphoteric surfactants because of their effectiveness and lower toxicity.

Various surfactants were tested during development in order to select the most proper one which will enhance the active ingredient's poor solubility. The surfactant used used in the present invention is polysorbate 80.

Preferably, polysorbate 80, is used in the present invention in an amount 0.2-4.5% (w/w). Most preferably polysorbate 80 is used in the present invention in an amount 2.5-3.5% (w/w).

The pharmaceutical compositions of the present invention may also contain one or more additional formulation excipients such as diluents, disintegrants, binders, lubricants, glidants provided that they are compatible with the active ingredient of the composition, so that they do not interfere with it in the composition and in order to increase the stability of the drug and the self-life of the pharmaceutical product. Diluents enhance flowability and compaction properties of the powder blend, increase the bulk of a solid pharmaceutical composition, and may make a pharmaceutical dosage form easier for the patient and care giver to handle. Diluents for solid compositions include, for example, microcrystalline cellulose (MCC), dextrose, fructose, mannitol, maltodextrin, maltitol, lactose.

The dissolution rate of a compacted solid pharmaceutical composition in the patient's stomach may be increased by the addition of a disintegrant to the composition. Disintegrants include sodium starch glycolate, alginic acid, carboxymethylcellulose sodium, croscarmelose sodium, crospovidone. More specifically, crospovidone due to its large swelling capacity in aqueous solution enhances the force needed to push particles apart within tablets pores exerted by the water, resulting in rapid tablet disintegration.

Solid pharmaceutical compositions that are compacted into a dosage form, such as a tablet, may include excipients whose function include helping to bind the active ingredient and other excipients together after compression. Binders for solid pharmaceutical compositions include hydroxyethyl cellulose, methylcellulose, hydroxypropyl cellulose (HPC), polydextrose, polyethylene oxide, povidone (PVP).

When a dosage form such as a tablet is made by the compaction of a powdered composition, the composition is subjected to pressure from a punch and dye. Some excipients and active ingredients have a tendency to adhere to the surfaces of the punch and dye, which can cause surface irregularities to the product. A lubricant can be added to the composition to reduce adhesion and ease the release of the product from the dye. Lubricants include talc, magnesium stearate, calcium stearate, glyceryl behenate, sodium stearyl fumarate.

Glidants are used to promote powder flow by reducing interparticle friction and cohesion. Glidants, may be, for example, colloidal silicon dioxide (Aerosil^{®}), calcium silicate, calcium phosphate tribasic.

Finally, the tablets of the present invention are optionally coated with a film composition that has no effect on the release of the active ingredient from the dosage form. The film-coating composition may comprise polymers, plasticizers, surfactants, opacifiers and/or pigments. The film-coating is prepared by dissolving the coating composition in water and/or organic solvent and then is sprayed on the tablet core. The coating process is commonly performed in a coating pan.

The following examples illustrate preferred embodiments in accordance with the present invention

### EXAMPLES

The development started with Composition 1 which was prepared with two different manufacturing processes to select the optimum one.

**Table 1: Composition 1**

| **Ingredients** | % |
|---|---|
| **Internal Phase** | |
| Deferasirox | 55,56 |
| MCC | 33,10 |
| PVP | 2,25 |
| Crospovidone | 7,00 |
| Poloxamer 188 | 0,10 |
| Aerosil | 0,50 |

| **External Phase** | |
|---|---|
| Magnesium Stearate | 1,50 |
| **Total for uncoated tablet** | 100,00 |

Composition 1 was first manufactured with wet granulation process using water as kneading solvent according to the following steps:
- Mixing API with MCC, PVP, Crospovidone, Poloxamer and Aerosil;
- Kneading with water and then drying at 40⁰C;
- Addition of Magnesium Stearate and mixing for 3min;
- Compression to tablets.

Composition 1 was also manufactured with a direct compression procedure comprising the following steps:
- Mixing API with MCC, PVP, Crospovidone, Poloxamer and Aerosil;
- Addition of Magnesium Stearate and mixing for 3min;
- Compression to tablets.

The physicochemical characteristics achieved are presented in the following table.

**Table 2: Results of Composition 1 manufactured with wet granulation & direct compression**

| **Composition 1** | | |
|---|---|---|
| | **Wet granulation** | **Direct compression** |
| **Hardness** | 162N | NA |
| **Disintegration** | 1'12" | NA |

Composition 1 manufactured with direct compression process was not tested further as the powder blend exhibited very bad flow properties (carr's index about 55%) due to the high drug load and the micronized form of the API
Composition 2 was prepared with lower tablet weight and higher API amount in the tablet. (Table 3)

**Table 3: Composition 2**

| **Ingredients** | % |
|---|---|
| **Internal Phase** | |
| Deferasirox | 66,18 |
| MCC | 23,23 |
| PVP | 2,00 |
| Crospovidone | 7,00 |
| Poloxamer 188 | 0,10 |
| Aerosil | 0,50 |

| **External Phase** | |
|---|---|
| Magnesium Stearate | 1,00 |
| **Total for uncoated tablet** | 100,00 |

Composition 2 was prepared according to the following manufacturing process:
- Mixing API with MCC, PVP, Crospovidone, Poloxamer and Aerosil;
- Kneading with water and then drying at 40⁰C;
- Addition of Magnesium Stearate and mixing for 3min;
- Compression to tablets.

The physicochemical characteristics achieved are presented in the following table.

**Table 4: Results of Composition 2**

| | **Composition 2** |
|---|---|
| **Hardness** | 131N |
| **Disintegration** | 1'12" |

Dissolution profiles of Composition 1 prepared with wet granulation process & Composition 2 are presented in table 5 below:

**Table 5: Dissolution in pH 6.8 + 0.5% Tween 20 75rpm**

| **Time (min)** | **Composition 1 (Wet granulation) % dissolved** | **Composition 2 % dissolved** |
|---|---|---|
| **5** | 56,90 | 54,63 |
| **10** | 72,41 | 71,20 |
| **15** | 79,22 | 79,04 |
| **20** | 82,96 | 83,85 |
| **30** | 88,59 | 88,19 |
| **45** | 92,84 | 91,80 |

As a next step different diluents were used in order to study their effect on dissolution properties and physical characteristics.

The following design of experiments with different diluent and different disintegrant in low and high amount was performed and tested according to the following table:

**Table 6: Design of experiments**

| | **Factors** | | | **Responses** | | |
|---|---|---|---|---|---|---|
| **Trial** | **Type of diluent** | **Type of disintegrant** | **Amount of disintegrant (%)** | **Hardness** | **Disintegration** | **% dissolved in 15min** |
| 3 | lactose | crospovidone | 3 | 102 | 88 | 86,62 |
| 4 | mannitol | crospovidone | 8 | 105 | 100 | 88,62 |
| 5 | mannitol | croscarmellose | 3 | 94 | 77 | 90,78 |
| 6 | mannitol | croscarmellose | 8 | 133 | 379 | 56,63 |
| 7 | mannitol | crospovidone | 3 | 120 | 95 | 68,17 |
| 8 | lactose | crospovidone | 8 | 99 | 61 | 92,17 |
| 9 | lactose | croscarmellose | 8 | 112 | 422 | 68,46 |
| 10 | lactose | croscarmellose | 3 | 109 | 149 | 83,29 |

From the design it was concluded that mannitol and crospovidone at higher concentration (about 8%) as diluent and disintegrant respectively exhibited better physical properties. Also a higher amount of disintegrant enhanced the dissolution properties.

However comparing Composition 2 with the experiments with mannitol it was concluded that MCC is necessary in the formulation to provide higher hardness.

Different surfactants were further studied as shown below in order to select the most suitable one in terms of physicochemical and dissolution properties of the final drug product. In addition, hydroxypropyl cellulose (HPC) was used as an alternative binder instead of PVP.

**Table 7: Composition 11-14**

| **Composition** | **11** | **12** | **13** | **14** |
|---|---|---|---|---|
| | **%** | | | |
| **Internal Phase** | | | | |
| Deferasirox | 66,18 | 66,18 | 66,18 | 66,18 |
| MCC | 22,12 | 22,12 | 22,12 | 22,12 |
| HPC | 2,00 | 2,00 | 2,00 | 2,00 |
| Crospovidone | 7,00 | 7,00 | 7,00 | 7,00 |
| Poloxamer | 0,50 | - | - | - |
| Polysorbate 80 | - | 0,50 | - | - |
| Sodium lauryl sulfate | - | - | 0,50 | - |
| Cremophor | - | - | - | 0,50 |
| Aerosil | 1,20 | 1,20 | 1,20 | 1,20 |

| **External Phase** | | | | |
|---|---|---|---|---|
| Magnesium Stearate | 1,00 | 1,00 | 1,00 | 1,00 |
| **Total for uncoated tablet** | 100,00 | | | |

Compositions 11-14 were prepared according to the following manufacturing process:
- Mixing API with MCC, HPC, Crospovidone, Aerosil and the respective surfactant;
- Kneading with water and then drying at 40⁰C;
- Addition of Magnesium Stearate and mixing for 3min;
- Compression to tablets.

The physicochemical characteristics achieved are presented in the following table.

**Table 8: Results of Compositions 11-14**

| **Composition** | **11** | **12** | **12** | **14** |
|---|---|---|---|---|
| **Hardness** | 188N | 138N | 134N | 150N |
| **Disintegration** | 2'11" | 1'18" | 2'30" | 2'50" |

Dissolution profiles of Compositions 11-14 are presented in table 9 below:

**Table 9: Dissolution in pH 6.8 + 0.5% Tween 20 75rpm**

| **Time (min)** | **Composition 11 % dissolved** | **Composition 12 % dissolved** | **Composition 13 % dissolved** | **Composition 14 % dissolved** |
|---|---|---|---|---|
| 5 | 53,06 | 37,38 | 53,47 | 40,08 |
| 10 | 71,67 | 73,80 | 66,57 | 65,23 |
| 15 | 75,03 | 81,62 | 72,98 | 75,05 |
| 20 | 81,86 | 85,28 | 77,62 | 80,98 |
| 30 | 88,19 | 91,83 | 84,48 | 83,35 |
| 45 | 92,23 | 94,76 | 88,33 | 88,72 |

Based on the results above the surfactant that showed the best behavior was polysorbate 80. Different amounts of polysorbate 80 were tested to determine the optimum range that is effective in terms of dissolution properties. Compositions 12 A-E with polysorbate 80 up to 4.5% w/w were prepared as below.

**Table 10: Compositions 12 A-E**

| **Composition** | **12A** | **12B** | **12C** | **12D** | **12E** |
|---|---|---|---|---|---|
| **Internal Phase** | **%** | | | | |
| Deferasirox | 66,18 | 66,18 | 66,18 | 66,18 | 66,18 |
| MCC | 22,42 | 21,62 | 20,12 | 19,12 | 18,12 |
| HPC | 2,00 | 2,00 | 2,00 | 2,00 | 2,00 |
| Crospovidone | 7,00 | 7,00 | 7,00 | 7,00 | 7,00 |
| **Polysorbate 80** | **0,20** | **1,00** | **2,50** | **3,50** | **4,50** |
| Aerosil | 1,20 | 1,20 | 1,20 | 1,20 | 1,20 |

| **External Phase** | | | | | |
|---|---|---|---|---|---|
| Magnesium Stearate | 1,00 | 1,00 | 1,00 | 1,00 | 1,00 |
| **Total for uncoated tablet** | 100,00 | | | | |

Compositions 12 A-E were prepared according to the following manufacturing process:
- Mixing API with MCC, HPC, Crospovidone, Aerosil and Polysorbate 80;
- Kneading with water and then drying at 40⁰C;
- Addition of Magnesium Stearate and mixing for 3min;
- Compression to tablets.

The physicochemical characteristics achieved are presented in the following table.

**Table 11: Results of Compositions 12 A-E**

| **Composition** | **12A** | **12B** | **12C** | **12D** | **12E** |
|---|---|---|---|---|---|
| **Hardness** | 164N | 147N | 131N | 108N | 94N |
| **Disintegration** | 1'10" | 1'45" | 3'40" | 4'00" | 6`00" |

Dissolution profiles of Compositions 12 A-E are presented in table 12 below:

**Table 12: Dissolution in pH 6.8 + 0.5% Tween 20 75rpm**

| **Time (min)** | **Composition 12A % dissolved** | **Composition 12B % dissolved** | **Composition 12C % dissolved** | **Composition 12D % dissolved** | **Composition 12E % dissolved** |
|---|---|---|---|---|---|
| 5 | 48,73 | 48,53 | 46,38 | 40,33 | 40,62 |
| 10 | 71,17 | 79,33 | 79,49 | 71,24 | 76,33 |
| 15 | 75,62 | 83,28 | 86,36 | 90,37 | 83,15 |
| 20 | 78,85 | 90,26 | 88,02 | 96,14 | 88,15 |
| 30 | 82,73 | 92,15 | 93,38 | 95,71 | 92,80 |
| 45 | 89,87 | 97,86 | 95,69 | 96,48 | 96,40 |

According to the results presented above, the amount of polysorbate 80 plays significant role in the dissolution properties of the tablet. Even though in terms of physical properties the increasing amount of polysorbate 80 results in tablets with lower hardness and higher disintegration time, the optimum range seems to be 2.5-4.0% w/w polysorbate 80 since it enhances the dissolution profile.

In an attempt to increase the tablet hardness, Composition 15 was prepared in which 3.5% polysorbate 80 and povidone (PVP) instead of HPC were used.

**Table 13: Composition 15**

| **Ingredients** | **%** |
|---|---|
| **Internal Phase** | |
| Deferasirox | 66,18 |
| MCC | 20,12 |
| PVP | 2,00 |
| Crospovidone | 7,00 |
| Polysorbate 80 | 3,50 |
| Aerosil | 1,20 |

| **External Phase** | |
|---|---|
| Magnesium Stearate | 1,00 |
| **Total for uncoated tablet** | 100,00 |

Composition 15 was prepared according to the following manufacturing process:
- Mixing API with MCC, PVP, Crospovidone, Aerosil and Polysorbate 80;
- Kneading with water and then drying at 40⁰C;
- Addition of Magnesium Stearate and mixing for 3min;
- Compression to tablets.

The physicochemical characteristics achieved are presented in the following table.

**Table 14: Results of Composition 15**

| | **Composition 15** |
|---|---|
| **Hardness** | 155N |
| **Disintegration** | 2'40" |

Dissolution profile of Composition 15 is presented in table 15 below:

**Table 15: Dissolution in pH 6.8 + 0.5% Tween 20 75rpm**

| **Time (min)** | **Composition 15 % dissolved** |
|---|---|
| 5 | 69,17 |
| 10 | 84,99 |
| 15 | 91,38 |
| 20 | 94,62 |
| 30 | 95,07 |
| 45 | 98,33 |

According to the results above the use of PVP instead of HPC improved the physical properties of the tablets and the initial time points in dissolution profile.

Due to the high drug load of the API and its flow properties some sticking issues were faced during compression to tablets so it was decided to include other lubricants in the formula as well. Apart from magnesium stearate that is included in Composition 15 also talc and sodium stearyl fumarate were used in Composition 16. Also the amount of PVP was further increased to optimize the tablet properties.

**Table 16: Composition 16**

| **Ingredients** | **%** |
|---|---|
| **Internal Phase** | |
| Deferasirox | 66,18 |
| MCC | 15,12 |
| PVP | 3,00 |
| Crospovidone | 7,00 |
| Polysorbate 80 | 3,50 |
| Aerosil | 1,20 |

| **External Phase** | |
|---|---|
| Talc | 1,50 |
| Sodium stearyl fumarate | 1,50 |
| Magnesium Stearate | 1,00 |
| **Total for uncoated tablet** | 100,00 |

Composition 16 was prepared according to the following manufacturing process:
- Mixing API with MCC, PVP, Crospovidone, Aerosil and Polysorbate 80;
- Kneading with water and then drying at 40⁰C;
- Addition of Talc, Sodium stearyl fumarate and Magnesium Stearate and mixing for 3min;
- Compression to tablets.

In order to optimize tablet characteristics an alternative process was evaluated. The preferred composition of the present invention is presented in table 17 below.

**Table 17: Composition 17**

| **Ingredients** | **%** |
|---|---|
| **Internal Phase** | |
| Deferasirox | 64.88 |
| Polysorbate 80 | 3.58 |
| PVP | 2.94 |
| Crospovidone | 4.90 |
| Purified water | |

| **Slugging Phase** | |
|---|---|
| Aerosil | 0.59 |
| Talc | 0.74 |
| Mg Stearate | 0.49 |

| **External Phase** | |
|---|---|
| MCC | 11.74 |
| Aerosil | 0.59 |
| Crospovidone | 4.90 |
| Talc | 0.74 |
| Sodium stearyl fumarate | 1.47 |
| Mg Stearate | 0.49 |
| **Total for uncoated tablet** | 98.04 |
| OPADRY II Blue | |
| Purified water | |
| **Total for coated tablet** | 100.00 |

The preferred composition of the present invention (Composition 17) is prepared according to the following manufacturing process:
- Wet granulation of internal phase excipients and API using water as kneading solvent;
- Drying;
- Mixing with slugging phase;
- Compressing into slugs;
- Milling the resulted slug to yield the granules;
- Mixing with external phase excipients;
- Tableting;
- Optionally applying a film-coating.

The physicochemical characteristics achieved are presented in the following table.

**Table 18: Results of Composition 17**

| | **Composition 17** |
|---|---|
| **Hardness** | 144N |
| **Disintegration** | 2'50" |

Dissolution profile of Composition 17 is presented in table 19 below:

**Table 19: Dissolution in pH 6.8 + 0.5% Tween 20 75rpm**

| **Time (min)** | **Composition 17 % dissolved** |
|---|---|
| 5 | 72,85 |
| 10 | 85,92 |
| 15 | 90,41 |
| 20 | 92,69 |
| 30 | 92,46 |
| 45 | 98,31 |

Since Composition 17 exhibited the optimum performance it was further film coated and tested for physical properties and dissolution.

The physicochemical characteristics achieved are presented in the following table.

**Table 20: Results of Composition 17 (included film-coating)**

| | **Composition 17 coated tablets** |
|---|---|
| **Hardness** | 175N |
| **Disintegration** | 4'10" |

Dissolution profile of Composition 17 including film-coating is presented in table 21 below:

**Table 21: Dissolution in pH 6.8 + 0.5% Tween 20 75rpm**

| **Time (min)** | **Composition 17 coated tablets (% dissolved)** |
|---|---|
| 5 | 57,11 |
| 10 | 83,03 |
| 15 | 88,93 |
| 20 | 89,25 |
| 30 | 95,46 |
| 45 | 99,23 |

In order to test the chemical stability of the API in the final product film-coated tablets were stored under normal and accelerated conditions for 6 months.

The stability data are presented in the following table:

**Table 22: Results of stability data under normal and accelerated conditions**

| | **6 MONTHS** | | |
|---|---|---|---|
| **SPECIFICATION** | **Zero time** | **25°C/60% RH** | **40°C/75%RH** |
| **Total impurities NMT 2.0%** | 0.03 | 0.04 | 0.04 |

## Claims

1. A solid pharmaceutical composition for oral administration comprising Deferasirox in an amount higher than 60% by weight based on the total weight of the medicament and polysorbate 80 to increase solubility and rate of dissolution.

2. The pharmaceutical composition according to claim 3, wherein polysorbate 80 is present in the formulation in the amount of 2.5-4.0% w/w.

3. The pharmaceutical composition according to any preceding claim comprising Deferasirox with particle size distribution of D90<10µm.

4. The pharmaceutical composition according to any preceding claim, wherein it further comprises at least one pharmaceutical acceptable excipient selected from diluents, binders, disintegrants, glidants and lubricants.

5. The pharmaceutical composition according to claim 4, wherein the lubricants are selected from talc, sodium stearyl fumarate and magnesium stearate.

6. The pharmaceutical composition according to any preceding claim, wherein it is in the form of film-coated tablets.

7. A process for the preparation of a tablet composition comprising Deferasirox in an amount higher than 60% by weight based on the total weight of the medicament and polysorbate 80 to increase solubility and rate of dissolution comprising the steps of:
- Wet granulation of API, Polysorbate 80, PVP and Crospovidone using water as kneading solvent;
- Drying;
- Mixing with slugging phase consisting of Aerosil, Talc and Magnesium stearate;
- Compressing into slugs;
- Milling the resulted slug to yield the granules;
- Mixing with MCC, Sodium stearyl fumarate and rest amount of Crospovidone, Aerosil, Talc and Magnesium stearate;
- Tableting;
- Optionally applying a film-coating.

8. The process according to claim 7, wherein polysorbate 80 is comprised in the amount of 2.5-4.0% w/w.

## Patentansprüche

1. Feste pharmazeutische Zusammensetzung zur oralen Verabreichung, die Deferasirox in einem Gewichtsverhältnis von mehr als 60% des Gesamtgewichts des Medikaments enthält sowie Polysorbat 80 zur Erhöhung der Löslichkeit und der Auflösungsgeschwindigkeit.

2. Pharmazeutische Zusammensetzung nach Anspruch 3, **dadurch gekennzeichnet, dass** Polysorbat 80 in einem Gewichtprozent von 2,5 bis 4,0% enthalten ist.

3. Pharmazeutische Zusammensetzung nach einem der vorhergehenden Ansprüche, **dadurch gekennzeichnet, dass** Deferasirox mit einer Partikelgröße von D90<10µm enthalten ist.

4. Pharmazeutische Zusammensetzung nach einem der vorhergehenden Ansprüche, **dadurch gekennzeichnet, dass** sie mindestens einen pharmazeutisch verwendbaren Hilfsstoff enthält, z.B. Spreng-/Zerfallsmittel, Bindemittel, Streckmittel, Gleitmittel und Schmiermittel.

5. Pharmazeutische Zusammensetzung nach Anspruch 4, **dadurch gekennzeichnet, dass** die Schmiermittel aus Talkum, Natriumstearylfumarat und Magnesiumstearat ausgewählt sind.

6. Pharmazeutische Zusammensetzung nach einem der vorhergehenden Ansprüche, **dadurch gekennzeichnet, dass** sie in der Form von Filmtabletten ist.

7. Verfahren für die Zubereitung einer Tablettenzusammensetzung, die Deferasirox in einem Gewichtsverhältnis von mehr als 60% des Gesamtgewichts des Medikaments enthält sowie Polysorbat 80 zur Erhöhung der Löslichkeit und der Auflösungsgeschwindigkeit, wobei das o.g. Verfahren die nachstehenden Schritte umfasst:
- Feuchtgranulation von API, Polysorbat 80, PVP und Crospovidon unter Verwendung von Wasser als Knetlösungsmittel;
- Trocknen;
- Mit der Slugging-Phase bestehend aus Aerosil, Talkum und Magnesiumstearat vermischen;
- In Rohlinge pressen;
- Die resultierenden Rohlinge mahlen, um Granulat zu erhalten;
- Mit MCC, Natriumstearylfumarat und die Restmenge von Crospovidon, Aerosil, Talkum und Magnesiumstearat vermischen;
- Tablettierung;
- Optional die Tabletten mit Film überziehen.

8. Verfahren nach Anspruch 7, **dadurch gekennzeichnet, dass** Polysorbat 80 in einem Gewichtprozent von 2,5 bis 4,0% enthalten ist.

## Revendications

1. Composition pharmaceutique solide pour administration orale comprenant du déférasirox en une quantité supérieure à 60 % en poids sur la base du poids total du médicament et du polysorbate 80 pour augmenter la solubilité et la vitesse de dissolution.

2. Composition pharmaceutique selon la 3ème revendication, dans laquelle le polysorbate 80 est présent dans la formulation en une quantité de 2,5 à 4,0 % p/p.

3. Composition pharmaceutique selon l'une quelconque des revendications précédentes, comprenant du déférasirox avec une distribution granulométrique de D90<10 µm.

4. Composition pharmaceutique selon l'une quelconque des revendications précédentes, qui comprend en outre au moins un excipient pharmaceutiquement acceptable choisi parmi les diluants, les liants, les désintégrants, les glissants et les lubrifiants.

5. Composition pharmaceutique selon la 4ème revendication, où sont choisis des lubrifiants parmi le talc, le fumarate de stéaryle de sodium et le stéarate de magnésium.

6. Composition pharmaceutique selon l'une quelconque des revendications précédentes, qui revêt la forme de comprimés pelliculés.

7. Procédé de préparation d'une composition de comprimé comprenant du déférasirox en une quantité supérieure à 60 % en poids par rapport au poids total du médicament et du polysorbate 80 pour augmenter la solubilité et la vitesse de dissolution, comprenant les étapes consistant à :
- la Granulation humide des substances actives, le Polysorbate 80, la PVP et la Crospovidone en utilisant de l'eau comme solvant de pétrissage ;
- le séchage,
- le Mélange avec phase de slugging consistant à l'Aérosil, le Talc et le Stéarate de Magnésium ;
- la Compression en limaces ;
- le Moulage de la limace résultante pour créer des granulés ;
- le Mélange avec le MCC, le fumarate de stéaryle de sodium et la quantité restante de crospovidone, d'aérosil, de talc et de stéarate de magnésium ;
- les Comprimés
- l'Application éventuelle d'un pelliculage.

8. Le procédé selon la 7ème revendication, dans lequel le polysorbate 80 est compris dans la quantité de 2,5 à 4,0 % p/p.
